(19) Europäisches Patentamt
European Patent Office
Office européen des brevets

(11) **EP 3 769 794 A1**

(12) **EUROPEAN PATENT APPLICATION**
published in accordance with Art. 153(4) EPC

(43) Date of publication:
27.01.2021 Bulletin 2021/04

(51) Int Cl.:
*A61L 9/14* (2006.01)     *A61L 9/22* (2006.01)
*A61L 2/14* (2006.01)     *C02F 1/48* (2006.01)

(21) Application number: 19856760.4

(22) Date of filing: 06.09.2019

(86) International application number:
PCT/CN2019/104746

(87) International publication number:
WO 2020/048531 (12.03.2020 Gazette 2020/11)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 07.09.2018 HK 18111547

(71) Applicant: Magic Water (Hong Kong) Limited
Hong Kong (CN)

(72) Inventors:
• LI, Junlu
  Hong Kong (CN)
• GAO, Qiang
  Hong Kong (CN)

(74) Representative: Isern Patentes y Marcas S.L.
Avda. Diagonal, 463 Bis, 2°
08036 Barcelona (ES)

(54) **STERILIZATION DEVICE**

(57) The disclosure relates to the technical field of sterilization and provides a sterilization device, wherein the sterilization device is arranged in a medium carrier circulating pipeline and is connected with a direct current power supply; the sterilization device comprises a medium electrolysis module arranged in the medium carrier circulating pipeline and used for realizing electrolysis or ionization of the medium carrier and increasing ion concentration in the medium carrier; a high-voltage generation module for converting the low-voltage direct current output by the direct current power supply into high-voltage direct current and acting on the electrolytic or ionized medium carrier to enable ions to have energy; and a feedback unit comprising a coil, wherein the feedback unit is respectively connected with the dielectric electrolysis module and the high-voltage generation module, and is used for detecting the feedback voltage generated when ions with energy pass through the coil, and performing closed-loop control according to the feedback voltage so as to adjust the electrolysis or ionization frequency of the dielectric electrolysis module and the output voltage of the high-voltage generation module in real-time. The application improves the utilization rate of the medium carrier and has a feedback adjustment function.

Figure 2

# Description

This application claims the priority of A STERILIZATION DEVICE with Hong Kong short-term patent application number 18111547.5 in the Intellectual Property Department of Hong Kong Special Administrative Region on September 7, 2018, and the disclosure is incorporated herein in its entirety by reference.

## Technical Field

The application relates to the field of sterilization and disinfection, in particular to a sterilization device.

## Background Technology

Sterilization refers to killing pathogenic bacteria in objects, while disinfection refers to killing pathogenic microorganisms, but not necessarily killing bacterial spores. In practical applications, sterilization and disinfection are often carried out simultaneously. Traditional sterilization methods include chemical disinfection, ultrasonic disinfection, ozone disinfection and ionized water disinfection.

Although water is used as the main sterilization carrier, which is green, safe and reliable to a certain extent. However, the traditional electrolytic water produces negative potential alkaline ion water and positive potential acidic ion water in the electrolysis process, but a part of acidic water is actually used for sterilization and disinfection, which will inevitably lead to the loss of alkaline water, resulting in the waste of water resources. In addition, due to the lack of feedback mechanism, sterilization and disinfection are output in one direction, so it cannot be adjusted in real-time according to the actual output situation to maintain stable sterilization and disinfection effect.

## Summary of the Invention

In order to solve the above technical problems, the embodiment of the present application provides a sterilization device, which improves the utilization rate of a medium carrier and has a feedback adjustment function.

To solve the above technical problems, the embodiment of this application provides the following technical scheme:

The embodiment of the application provides a sterilization device, which is arranged in a pipeline through which a medium carrier circulates and is connected with a direct current power supply. The sterilization device comprises:

A medium electrolysis module is arranged in the pipeline through which the medium carrier circulates, and is used for realizing electrolysis or ionization of the medium carrier and increasing ion concentration in the medium carrier;

A high-voltage generation module is used for converting the low-voltage direct current output by the direct current power supply into high-voltage direct current and acting on the dielectric carrier after electrolysis or ionization to energize the ions; and

A feedback unit comprises a coil and is respectively connected with the dielectric electrolysis module and the high-voltage generation module, and is used for detecting the feedback voltage generated when ions with energy pass through the coil, and performing closed-loop control according to the feedback voltage, thereby adjusting the electrolysis or ionization frequency of the dielectric electrolysis module and the output voltage of the high-voltage generation module in real-time.

Furthermore, the sterilization device comprises a high-voltage access unit and a metal adapter, wherein the high-voltage access unit is respectively connected with the high-voltage generation module and the metal adapter, and the metal adapter is used for conducting the output voltage of the high-voltage generation module into the medium carrier through the high-voltage access unit.

Furthermore, an electrolytic anode and an electrolytic cathode are arranged in the pipeline through which the medium carrier flows, and an ion exchange membrane is arranged between the electrolytic anode and the electrolytic cathode;

The medium electrolysis module comprises a boosting circuit, a first switch circuit and a first control circuit;

The boosting circuit is connected with the DC power supply and used for amplifying the voltage input by the DC power supply into the boosting circuit and outputting the amplified voltage;

The first switch circuit is connected with the boosting circuit and receives an output voltage of the boosting circuit; the first switch circuit is respectively connected with the electrolytic anode and the electrolytic cathode and is used for outputting a positive voltage on the electrolytic anode and a negative voltage on the electrolytic cathode;

The first control circuit is respectively connected with the boosting circuit and the first switch circuit and is used for adjusting the on-time of the boosting circuit, the on-time of the anode voltage and the cathode voltage in one cycle.

Furthermore, the first control circuit is a DSP, and the on-time of the boosting circuit has a proportional coefficient relationship with the feedback voltage in one cycle, and the on-time of the negative voltage has a proportional coefficient relationship with the feedback voltage in one cycle.

Furthermore, the high voltage generation module includes a second control circuit, a second switch circuit and a transformer;

The second control circuit is connected with the second switch circuit and used for adjusting the duty ratio of the second switch circuit;

The second switch circuit is connected with the DC power supply and used for controlling the turn-on and turn-off of the high-voltage generating module;

The transformer is connected with the second switch circuit and used for converting low-voltage direct current output by the direct current power supply into high-voltage direct current.

Furthermore, the sterilization device also comprises a driving unit and an atomizing nozzle;

The driving unit is connected with a water pump or an air pump arranged in a pipeline through which the medium carrier flows;

The feedback unit is arranged on the atomizing nozzle, and the metal adapter is arranged in the pipeline in front of the atomizing nozzle through which the medium carrier flows.

Furthermore, the feedback unit further comprises a current detection circuit which is connected with the coil and used for detecting the current flowing through the coil.

Furthermore, the current detection circuit is arranged in the pipeline through which the medium carrier flows between the metal adapter and the atomizing nozzle, and is used for calculating the current flowing through the coil by detecting the carrier concentration and carrier velocity of the medium carrier.

Furthermore, the boosting circuit comprises an inductor L, a triode Q, a diode D, a capacitor C and a resistor R.:

One end of the inductor L is connected with the anode of the DC power supply, and the other end of the inductor L is connected with the collector of the triode Q;

The base of the triode Q is connected with the first control circuit, and the emitter of the triode Q is connected with the cathode of the DC power supply.

The anode of the diode D is connected with the collector of the triode Q, and the cathode of the diode D is connected with one end of the capacitor C;

The other end of the capacitor c is connected with the cathode of the DC power supply;

One end of the resistor R is connected with one end of the capacitor C, and the other end of the resistor R is connected with the other end of the capacitor C.

The embodiment of the application also provides sterilization equipment, which comprises the sterilization device described above, wherein the sterilization device is embedded in the sterilization equipment.

The advantage of this application is that compared with the prior art, the embodiment of the present application provides a sterilization device. Ion concentration in the medium carrier is increased through the medium electrolysis module, so the conductivity of the medium carrier is increased, and ions in the medium carrier are brought with high voltage under the action of the high voltage generation module. Therefore, both positive ions and negative ions in the medium carrier can act on cell membranes of microorganisms, thereby realizing sterilization and disinfection effect and improving the utilization rate of the medium carrier. At the same time, the feedback unit detects the feedback voltage generated when ions with energy pass through the coil and perform closed-loop control according to the feedback voltage, thereby adjusting the electrolysis or ionization frequency of the medium electrolysis module and the output voltage of the high-voltage generation module in real-time, and finally adjusting the potential difference acting on the cell membrane of microorganisms so that the cell membrane generates irreversible puncture holes, which destroys the metabolism of microorganisms and realizes sterilization and disinfection. As a result, the feedback unit can make the sterilization device maintain stable and keep an efficient sterilization effect.

## Description of Drawings

One or more embodiments are illustrated by the corresponding drawings, which do not constitute a limitation of the embodiments. Elements with the same reference numerals in the drawings are represented as similar elements, and unless otherwise stated, the drawings in the drawings do not constitute a scale limitation.

Fig. 1 is a structural schematic diagram of a sterilization device provided by an embodiment of the application;

Fig. 2 is a structural schematic diagram of a sterilization device provided by an embodiment of the application;

Fig. 3 is a structural schematic diagram of a sterilization device provided by another embodiment of the application;

Fig. 4 is a schematic structural diagram of the dielectric electrolysis module 10 in Fig. 3;

Fig. 5 is a schematic structural diagram of the boosting circuit 101 in Fig. 4;

Fig. 6 is a schematic structural diagram of the high voltage generation module 20 in Fig. 3;

Fig. 7 is a schematic diagram of the magnetic field distribution of the current-carrying circular coil provided by an embodiment of the application;

Fig. 8 is a schematic diagram of the electric field distribution of the current-carrying circular coil provided by an embodiment of the application.

## Embodiments

In order to facilitate the understanding of the application, the application is described in more detail with reference to the drawings and specific embodiments. It should be noted that when an element is said to be "connected" to another element, it may be directly connected to another element, or there may be one or more intermediate elements in between. As used in this specification, the terms "vertical", "horizontal", "left", "right", "upper", "lower", "inner" and "outer" indicate the orientation or positional relationship based on the orientation or positional relationship shown in the drawings, which is only for the convenience of describing the application and simplifying the description, but does not indicate or imply that the referred device or element must have a specific orientation, a specific position or operation, so it cannot constitute a limitation for this application. In addition, terms such as "first" and "second" are only used for descriptive purposes and cannot be understood as indicating or implying relative importance.

Unless otherwise defined, all technical terms used in this specification have the same meanings as those commonly understood by the skilled in the technical field. Terms used in the specification of this application are only for the purpose of describing specific embodiments and are not to limit this application. As used in this specification, the term "and/or" includes any and all combinations of one or more related listed items.

In addition, the technical features involved in different embodiments of the application described below can be combined with each other as long as they do not conflict with each other.

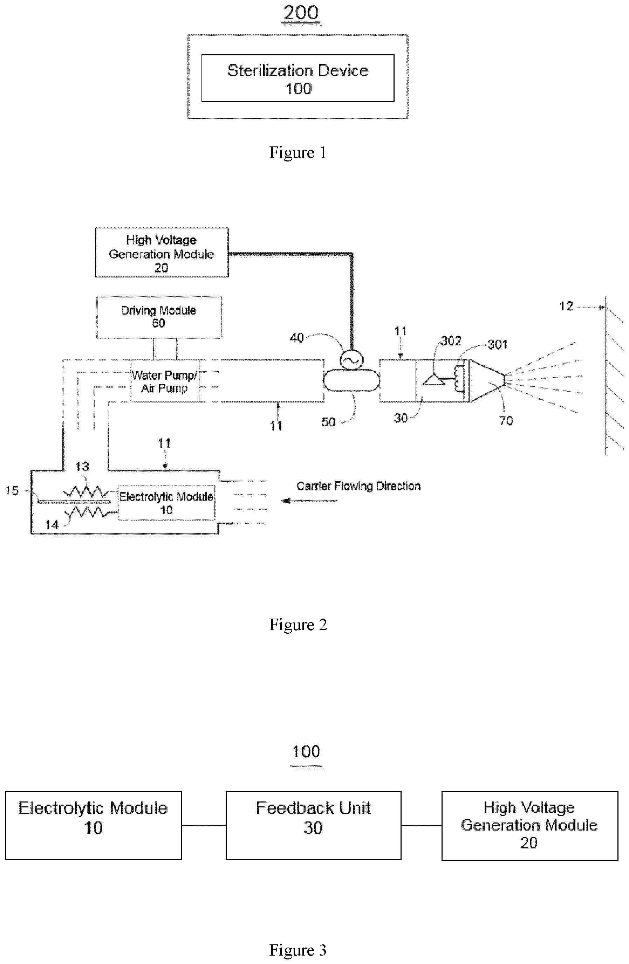

Fig. 1 is a structural schematic diagram of the sterilization device provided by the embodiment of the application. As shown in Fig. 1, the sterilization equipment 200 includes a sterilization device 100, wherein the sterilization device 100 is embedded in the sterilization equipment 200. It should be noted that the embedding can be realized by structural clamping, welding, locking screws, etc. The sterilization device 100 can be fixedly installed or detachably installed in the sterilization equipment 200.

In the embodiment of this application, the sterilization equipment 200 can be a sprayer, an intelligent toilet cover, a faucet, a bathroom product, a sweeper, various small fans, etc., and is used for killing microorganisms such as bacteria on the surface of an object or skin through a medium carrier. For example, when the sterilization equipment 200 is a bathroom product, the medium carrier is water; and when the sterilization apparatus 200 is a small fan, the medium carrier is air.

It can be seen that the sterilization equipment 200 does not need to introduce additional substances (such as chemical disinfectants and ultrasonic waves) for sterilization and disinfection, and the medium carrier is necessary for the working process of the sterilization equipment 200. For example, water is an essential element for the normal operation of faucets or bathroom products, and the air is used by sweepers and small fans to make the internal fans run during normal operation. The specific medium carrier used depends on the working principle of the sterilization device 200, which significantly reduces the manufacturing and transportation costs. In addition, no matter which medium carrier is used, the final product is water or air, which will not cause secondary pollution and damage to sterilized objects (such as clothes and skin surfaces).

It should be noted that because the sterilization device 100 is embedded in the sterilization equipment 200, the sterilization device 100 can be of modular general design, which is convenient to be embedded in common hand-held sterilizers, vegetable washers, faucets, smart toilet covers, and water spraying units of bathroom or dryers. The sterilization device 100 has a wide application range and is not limited to a single type of product. In terms of electrical performance (including rated voltage, rated current and output power, etc.) and size of the sterilization device 100, it can be fixed to adapt to different designs, or it can be adapted to the sterilization device 100 by changing relevant parameters.

In specific applications, the sterilization device 200 can be aimed at an area to be sterilized and disinfected, and the sterilization device 200 can be started to take effect on the surface of an object to be sterilized and disinfected. The sterilization device 200 can be started by triggering a start button set on the surface of the sterilization device 200 or a start option on a display screen. In some embodiments, the sterilization device 200 is remotely connected with a server, and the sterilization device 200 can be started through a dedicated APP on an intelligent terminal. Furthermore, the function of timing sterilization can be started, or the working time (including start time and close time) of the sterilization device 200 can be set. Therefore, the peak period of other functions of the sterilization device 200 can be effectively avoided and the user experience is improved.

In the Figs. 2 and 3, the sterilization device 100 includes a medium electrolysis module 10, a high-voltage generation module 20, a feedback unit 30, a high-voltage access unit 40, a metal adapter 50, a driving unit 60 and an atomizing nozzle 70. In the embodiment of this application, the sterilization device 100 is arranged in a pipeline 11 through which the medium carrier circulates, connected with a DC power supply, and acts on the surface 12 of an object to be sterilized, wherein the surface 12 of the object to be sterilized can be a vegetable surface, an outer wall of a toilet, human skin and the like. An electrolytic positive electrode 13 and an electrolytic negative electrode 14 are arranged in the pipeline 11 through which the medium carrier flows, and an ion exchange membrane 15 is arranged between the electrolytic positive electrode 13 and the electrolytic negative electrode 14. The DC power supply can be a built-in power supply (such as a storage battery) of the sterilization device 200, or an external DC power supply for providing power for the sterilization device 100.

Preferably, a water pump/air pump is arranged in the pipeline 11 through which the medium carrier circulates, which can be used for lifting and conveying water/air or increasing the pressure of water/air, that is, using the mechanical energy generated by a Prime Mover to achieve the purpose of pumping water/air. In some embodiments, the water pump/air pump can be omitted, and the driving unit 60 for driving the water pump/air pump can also be omitted accordingly.

It can be understood that the pipeline 11 through which the medium carrier flows can be a solid pipeline, for example, a pipeline of a device for conveying gas, liquid, or fluid with solid particles, which is connected by pipes, pipe couplings and valves. The pipeline can be made of plastic or metal, and preferably, the medium carrier does not contain impurities (i.e. solid particles). The medium carrier circulation pipe 11 can also be a virtual pipe, for example, a gap or space where the medium carrier can circulate, with no solid pipeline for guiding. In the embodiment of the present application, the medium carrier can be water and air, and can also be an ionizable or electrolytic media, such as Cucl2 solution, Fecl3 solution and the like.

The sterilization device 100 can be installed in all or part of the medium carrier circulation pipeline 11. In this embodiment, the medium electrolysis module 10, the feedback unit 30 and the metal adapter 50 are arranged in the medium carrier circulation pipeline 11 to contact with the medium carrier, while other modules and units are arranged outside the medium carrier circulation pipeline 11 and connected with those arranged in the medium carrier circulation pipeline 11.

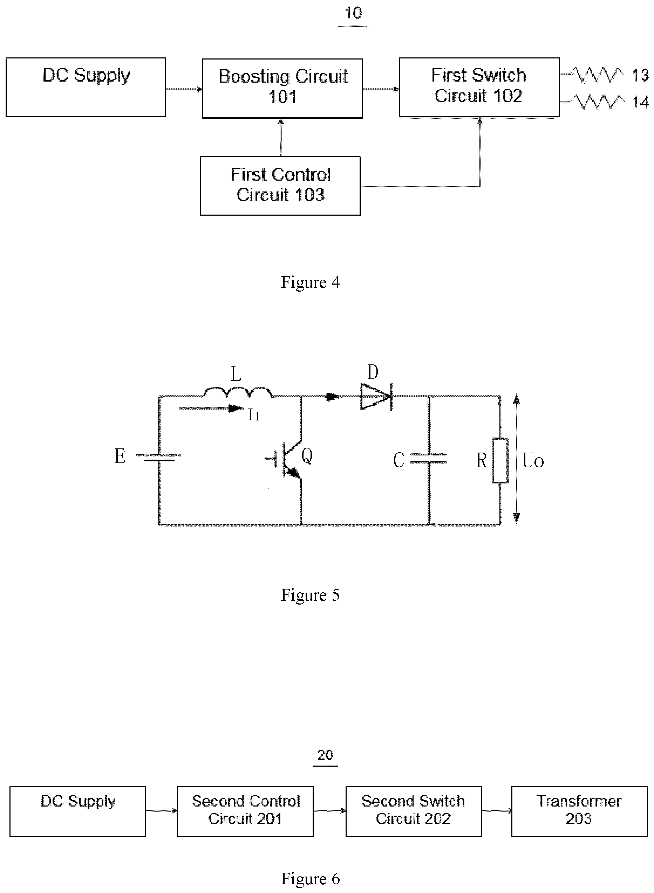

In Fig. 4, the dielectric electrolysis module 10 is arranged in the pipeline 11 through which the dielectric carrier circulates, and is used for realizing electrolysis or ionization of the dielectric carrier and increasing the ion concentration in the dielectric carrier. The dielectric electrolysis module 10 includes a boosting circuit 101, a first switch circuit 102 and a first control circuit 103.

The boosting circuit 101 is connected to the DC power supply and is used to amplify and output the voltage of the boosting circuit 101 from the DC power supply driven by the first control circuit 103.

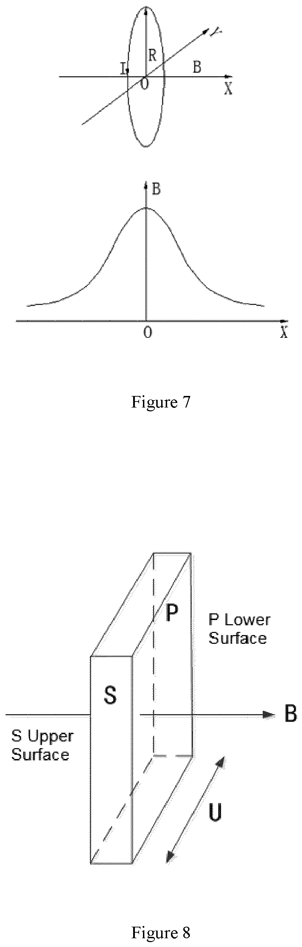

In Fig. 5, the boosting circuit 101 includes an inductor L, a triode Q, a diode D, a capacitor C and a resistor R, wherein one end of the inductor L is connected with the anode of the DC power supply, the other end of the inductor L is connected with the collector of the triode Q, the base of the triode Q is connected with the first control circuit, and the emitter of the triode Q is connected with the cathode of the DC power supply. The anode of the diode D is connected with the collector of the triode Q; the cathode of the diode D is connected with one end of the capacitor C, and the other end of the capacitor C is connected with the cathode of the DC power supply, and one end of the resistor R is connected with one end of the capacitor C, and the other end of the resistor R is connected with the other end of the capacitor C. The triode Q is the control switch tube of the boosting circuit 101, which is driven by PWM provided by the first control circuit, and the duty ratio is determined by the feedback voltage U (as shown in Fig. 8).

When the triode Q is in on state, the DC power supply (assuming the voltage is E) charges the inductor L, the charging current is constant I1, the voltage on the capacitor C supplies power to the resistor (i.e. the load), and its basic output voltage is constant Uo. Assuming that the on-time of the triode Q is ton, the energy accumulated on the inductor is E*I1*ton, when the triode Q is turned off, the DC power supply and the inductor L charge the capacitor C at the same time, and the voltage on the capacitor C supplies power to the resistor. Assuming that the turn-off time of the triode Q is toff, the energy released by the inductor is (Uo-E)*I1*toff. In a period T, the energy accumulated by the inductor L is equal to the energy released by the inductor L, that is, E*I1*ton=(Uo-E)*I1*toff, which is simplified to Uo=T/toff* E, so the amplification factor of the boost circuit 101 is T/toff.

The boosting circuit 101 discloses a Boost structure. It can be understood that the boosting circuit 101 can also be other forms of boost circuits, modules or integrated IC, or the boost circuit 101 can also be a step-up and step-down circuit, which can adjust the output voltage of the boosting circuit 101 in a larger range and more accurately.

The first switch circuit 102 is connected to the boosting circuit 101 and receives the output voltage of the boosting circuit 101. The first switch circuit 102 is respectively connected to the electrolytic positive electrode 13 and the electrolytic negative electrode 14 and is used for outputting positive voltage on the electrolytic positive electrode 13 and negative voltage on the electrolytic negative electrode 14.

The first control circuit 103 is respectively connected with the boost circuit 101 and the first switch circuit 102 and is used for adjusting the on-time of the boost circuit 101, the on-time of the anode voltage and the on-time of the cathode voltage in one cycle. In this embodiment, the first control circuit 103 is a DSP, and the on-time of the boost circuit 101 is proportional to the feedback voltage U in one cycle, and the on-time of the negative voltage is proportional to the feedback voltage U in one cycle.

In this embodiment, the first switching circuit 102 uses IR2103 to drive MOS, and the first switch circuit 102 inverts to generate AC square wave, which is applied to the dielectric carrier in the pipeline where the dielectric electrolysis module 10 is located, and the concentration of ions in the dielectric carrier is increased by electrolysis. The AC square wave signal will have a short electrode transition. It is assumed that the on-time of the boost circuit 101 is ton in one cycle. The turn-on time of the anode voltage in one cycle is Tdjon, and the turn-on time of the cathode voltage in one cycle is Tdjoff, so ton=Kf1U, Kf1 is the first feedback coefficient. Preferably, Tdjon: Tdjoff = 0.9: 0.1, Tdjoff= Kf2U, Kf2 is the second feedback coefficient. The effect of electrolysis (i.e., the concentration of ions in the medium carrier) can be adjusted by changing the frequency of electrolysis (i.e., the ratio of Tdjon to Tdjoff).

It can be understood that the medium electrolysis module 10 is designed for the medium carrier of liquid, and when the medium carrier is air, the medium electrolysis module 10 can be omitted. When the medium is water, the dielectric electrolysis module 10 increases the conductivity of water by increasing the ion concentration in the water. Driven by the water pump, the water flowing through the dielectric electrolysis module 10 (the water flowing through the pipeline where the dielectric electrolysis module 10 is installed) is partially ionized so that positive and negative ions with a certain concentration appear at the electrolytic anode 13 and the electrolytic cathode 14. When the medium is air, because the conductivity of air is poor, it is necessary to increase the voltage amplitude by voltage doubling. The voltage doubling DC output unit is connected to the output end of the transformer 203 (as shown in Fig. 6), which can realize multi-stage voltage doubling and output a negative pressure or AC high voltage. The ionized air is transmitted to the cell membrane of microorganisms (such as bacteria), thus achieving the sterilization effect.

In Fig. 6, the high-voltage generation module 20 is used to convert the low-voltage DC power output by the DC power supply into high-voltage DC power and act on the dielectric carrier after electrolysis or ionization to energize the ions. The high-voltage generation module 20 includes a second control circuit 201, a second switch circuit 202 and a transformer 203. The second control circuit 201 is connected with the second switch circuit 202 for adjusting the duty ratio of the second switch circuit 202, the second switch circuit 202 is also connected with the DC power supply for controlling the on and off of the high voltage generation module 20, and the transformer 203 is connected with the second switch circuit 202 for converting the low voltage DC output by the DC power supply into high voltage DC.

In this embodiment, the output voltage of the DC power supply is adjustable. The second switching circuit 202 uses IR2103S to drive MOS, and its switching frequency is usually 10 kHz-40 kHz. According to the coil turns ratio of the transformer 203, the output voltage of the DC power supply can be converted into a high voltage sine wave of 1500V-2000V or above.

The feedback unit 30 includes a coil 301, which is respectively connected with the dielectric electrolysis module 10 and the high-voltage generation module 20. The feedback unit 30 is used for detecting the feedback voltage U generated when ions with energy pass through the coil 301, and performing closed-loop control according to the feedback voltage U, thereby adjusting the electrolysis or ionization frequency of the dielectric electrolysis module 10 and the output voltage of the high-voltage generation module 20 in real-time.

The feedback unit 30 further includes a current detection circuit 302, which is connected with the coil 301 and used for detecting the current flowing through the coil 301. The current detection circuit 302 is disposed of in the pipeline 11 through which the medium carrier flows between the metal adapter 50 and the atomizing nozzle 70, and is specifically used to calculate the current flowing through the coil 301 by detecting the carrier concentration and carrier velocity of the medium carrier. In this embodiment, the current detection circuit 302 can be AD8217 current detection chip, and calculate the feedback voltage U according to the relationship between current and resistance.

In Fig. 7 and Fig. 8, according to ampere's loop rule and Faraday's electromagnetic induction law, ions with energy passing through the closed coil will generate induced electromotive force on the coil 301, and the magnetic induction intensity B of a current-carrying circular coil at a certain point on the axis (a straight line passing through the center of the circle and perpendicular to the coil):

$$B = \frac{\mu_0 N_0 I R^2}{2(R^2 + x^2)^{3/2}}$$

Wherein, $\mu_0 = 4\pi \times 10^{-7}$ H/m is magnetic permeability, R is the average radius of the coil, $N_0$ is the number of turns of the circular coil, I is the distance from a certain point on the axis to the center 0, and x is the current passing through the coils. Coils are two identical circular coils, which are parallel and coaxial with each other, and are supplied with current I in the same direction. Theoretical calculation proves that when the coil spacing A is equal to the coil radius R, the combined magnetic field of the two coils is uniform in a relatively large range near the axis (i.e., the connecting line between the centers of the two coils). It can be understood that the winding of the coil 301 should be as close as possible to the inner wall of the pipe 11 through which the medium carrier flows, and generally it can be wound for 3-5 turns.

Ions with energy move under the action of an electric field, which in turn prevents the ions with energy from continuing to deviate to the upper surface S. When Lorentz force and reaction force of the electric field are balanced, the ions with energy cannot deviate to the upper plane S. At this time, a stable voltage, namely the feedback voltage U, is formed between the upper surface S and the lower surface P. The coil 301 have a length 1, a width b, a thickness d, a carrier concentration n and a carrier velocity v, and the current I passing through the coil 301 is:

$I = nevbd$

The feedback voltage is: $U = IB / ned = R_H IB / d = K_H IB$

Wherein, the feedback coefficient is $R_H = 1/ne$, the unit is $m^3/C$; feedback sensitivity $K_H = R_H / d$, in mV/mA. Therefore, when I is constant, $U = K_H IB = k_0 B$ the closed-loop control can be performed in the sterilization device 100 by measuring the feedback voltage U.

The high-voltage access unit 40 is respectively connected with the high-voltage generating module 20 and the metal adapter 50. In this embodiment, the high-voltage access unit 40 includes a wire connecting the high-voltage generation module 20 and a connection point with the metal adapter 50, and the high-voltage access unit 40 and the metal adapter 50 can be connected and fixed by welding or locking screws.

The metal adapter 50 is used to conduct the output voltage of the high voltage generating module 20 to the dielectric carrier through the high voltage access unit 40. Preferably, the metal adapter 50 is made of copper. It can be understood that the metal adapter 50 can also be made of other metals with excellent conductivity.

The driving unit 60 is connected with a water pump or an air pump arranged in the pipeline 11 through which the medium carrier flows, and is used for driving the water pump or the air pump.

The feedback unit 30 is arranged on the atomizing nozzle 70, and the metal adapter 50 is arranged in the pipeline 11 in front of the atomizing nozzle 70 through which the medium carrier flows. The atomizing nozzle 70 can be understood as an outlet of water or air. When the sterilization device 200 has an outlet with corresponding functions, it is not necessary to set the atomizing nozzle 70, which increases the spraying area of water or air and enlarges the sterilization and disinfection area.

During application, the atomizing nozzle 70 is aimed at the surface 12 of the sterilized object, and the distance between the atomizing nozzle 70 and the surface 12 of the sterilized object is adjusted according to the output intensity of water or air with high potential ions, generally keeping a distance of several centimeters to tens of centimeters. Ionized water spray or ion air spray acts on the surface 12 of the sterilized object to form conductive gas-liquid mixed liquid on the surface 12 of the sterilized object so that the microorganisms on the surface 12 of the sterilized object are suspended therein, and the high voltage electric field acts on the microorganisms in the mixed liquid. Under the action of high voltage electric field, the electric charges on the cell membrane are separated to form a potential difference, which makes irreversible breakdown holes formed on the cell membrane, thus destroying the metabolism of microorganisms, leading to their death and realizing sterilization and disinfection.

The embodiment of the application provides a sterilization device and sterilization equipment, wherein the sterilization device increases the ion concentration in a medium carrier through a medium electrolysis module so that the conductivity of the medium carrier is increased, and the ions in the medium carrier are brought with high voltage under the action of a high-voltage generation module, so that both positive ions and negative ions in the medium carrier can act on cell membranes of microorganisms, thereby realizing sterilization and disinfection effects and improving the utilization rate of the medium carrier. Meanwhile, the feedback unit detects the feedback voltage generated when ions with energy pass through the coil and perform closed-loop control according to the feedback voltage, thereby adjusting the electrolysis or ionization frequency of the medium electrolysis module and the output voltage of the high-voltage generation module in real-time, and finally adjusting the potential difference acting on the cell membrane of microorganisms so that the cell membrane generates irreversible puncture holes, which destroys the metabolism of microorganisms and realizes sterilization and disinfection. As a result, the feedback unit can make the sterilization device maintain stable and keep an efficient sterilization effect.

It should be noted that the above embodiments are only used to illustrate the technical scheme of this application, but not to limit it. According to the technology of this application, the technical features in the above embodiments or different embodiments can also be combined, the steps can be realized in any order, and there are many other changes in different aspects of this application as mentioned above, which are not provided in details for brevity. Although the application has been described in detail with reference to the foregoing embodiments, those of ordinary skill in the art should understand that the technical solutions described in the foregoing embodiments can still be modified or some of the technical features can be replaced equivalently. However, these modifications or substitutions do not make the essence of the corresponding technical solutions deviate from the scope of the technical solutions of each embodiment of this application.

**Claims**

1. A sterilization device is **characterized in that** the sterilization device is arranged in a pipeline through which a medium carrier flows and is connected with a direct current power supply, wherein the sterilization device comprises:

   A medium electrolysis module, which is arranged in a pipeline through which the medium carrier circulates, and is used for realizing electrolysis or ionization of the medium carrier and increasing ion concentration in the medium carrier;

   A high-voltage generation module, which is used for converting the low-voltage direct current output by the direct current power supply into high-voltage direct current and acting on the dielectric carrier after electrolysis or ionization to energize the ions; and

   A feedback unit, which comprises a coil and is respectively connected with the dielectric electrolysis module and the high-voltage generation module, and is used for detecting the feedback voltage generated when ions with energy pass through the coil, and performing closed-loop control according to the feedback voltage, thereby adjusting the electrolysis or ionization frequency of the dielectric electrolysis module and the output voltage of the high-voltage generation module in real-time.

2. According to claim 1, the sterilization device further comprises a high-voltage access unit and a metal adapter, wherein the high-voltage access unit is respectively connected with the high-voltage generating module and the metal adapter, and the metal adapter is used for conducting the output voltage of the high-voltage generating module to the medium carrier through the high-voltage access unit.

3. According to claim 1, the sterilization device is **characterized in that** an electrolytic anode and an electrolytic cathode are arranged in the pipeline through which the medium carrier flows, and an ion exchange membrane is arranged between the electrolytic anode and the electrolytic cathode;
The medium electrolysis module comprises a boosting circuit, a first switch circuit and a first control circuit;
The boosting circuit is connected with the DC power supply and used for amplifying the voltage input by the DC power supply into the boosting circuit and outputting the amplified voltage;
The first switch circuit is connected with the boosting circuit and receives an output voltage of the boosting circuit, and is respectively connected with the electrolytic anode and the electrolytic cathode, and is used for outputting a positive voltage on the electrolytic anode and a negative voltage on the electrolytic cathode;
The first control circuit is respectively connected with the boost circuit and the first switch circuit and is used for adjusting the on-time of the boost circuit, the on-time of the anode voltage and the on-time of the cathode voltage in one cycle.

4. According to claim 3, the first control circuit is a DSP, and the on-time of the boost circuit is proportional to the feedback voltage in one cycle, and the on-time of the negative voltage is proportional to the feedback voltage in one cycle.

5. According to claim 1, the high voltage generation module comprises a second control circuit, a second switch circuit and a transformer;
The second control circuit is connected with the second switch circuit and used for adjusting the duty ratio of the second switch circuit;
The second switch circuit is also connected with the DC power supply, and is used for controlling the turn-on and turn-off of the high-voltage generating module;
The transformer is connected with the second switch circuit and used for converting low-voltage direct current output by the direct current power supply into high-voltage direct current.

6. According to claim 2, the sterilization device further comprises a driving unit and an atomizing nozzle;
The driving unit is connected with a water pump or an air pump arranged in a pipeline through which the medium carrier flows;
The feedback unit is arranged on the atomizing nozzle, and the metal adapter is arranged in the pipeline in front of the atomizing nozzle through which the medium carrier flows.

7. According to claim 6, the feedback unit further comprises a current detection circuit connected with the coil, and the current detection circuit is used for detecting the current flowing through the coil.

8. According to claim 7, the current detection circuit is arranged in a pipeline through which the medium carrier flows between the metal adapter and the atomizing nozzle, and is specifically used for calculating the current flowing through the coil by detecting the carrier concentration and carrier velocity of the medium carrier.

9. According to claim 3, the boosting circuit comprises an inductor L, a triode Q, a diode D, a capacitor C and a resistor R;
One end of the inductor L is connected with the anode of the DC power supply, and the other end of the inductor L is connected with the collector of the triode Q;
The base of the triode Q is connected with the first control circuit, and the emitter of the triode Q is connected with the cathode of the DC power supply.
The anode of the diode D is connected with the collector of the triode Q, and the cathode of the diode D is connected with one end of the capacitor C;
The other end of the capacitor C is connected with the cathode of the DC power supply; One end of the resistor R is connected with one end of the capacitor C, and the other end of the resistor R is connected with the other end of the capacitor C.

## 200

Sterilization Device
100

Figure 1

High Voltage
Generation Module
20

Driving Module
60

40

11

302   301

12

Water Pump/
Air Pump

11

30

50

70

11

13

15

Electrolytic Module
10

Carrier Flowing Direction

14

Figure 2

## 100

| Electrolytic Module 10 | Feedback Unit 30 | High Voltage Generation Module 20 |
|---|---|---|

Figure 3

10

| DC Supply | → | Boosting Circuit 101 | → | First Switch Circuit 102 | ⌇⌇ 13 ⌇⌇ 14 |
|---|---|---|---|---|---|

First Control Circuit 103

Figure 4

E ⊣⊢    L    $I_1$    Q    D    C ⊣⊢    R    Uo

Figure 5

20

| DC Supply | → | Second Control Circuit 201 | → | Second Switch Circuit 202 | → | Transformer 203 |
|---|---|---|---|---|---|---|

Figure 6

Figure 7

Figure 8

## INTERNATIONAL SEARCH REPORT

| International application No. |
| --- |
| **PCT/CN2019/104746** |

**A.    CLASSIFICATION OF SUBJECT MATTER**

A61L 9/14(2006.01)i;  A61L 9/22(2006.01)i;  A61L 2/14(2006.01)i;  C02F 1/48(2006.01)i

According to International Patent Classification (IPC) or to both national classification and IPC

**B.    FIELDS SEARCHED**

Minimum documentation searched (classification system followed by classification symbols)

C02F; A61L; B05C

Documentation searched other than minimum documentation to the extent that such documents are included in the fields searched

Electronic data base consulted during the international search (name of data base and, where practicable, search terms used)

EPODOC; CNKI; VEN; CNTXT; CNABS; Elsevier Science: 杀菌, 灭菌, 消毒, 电解, 电离, 离子, 高压, 反馈, 线圈, 检测, steriliz+, antibacteria, saniti+, disinfect+, electro+, ioniz+, plasma, ion, high voltage, feedback, coil?, detect+, monitor+

**C.    DOCUMENTS CONSIDERED TO BE RELEVANT**

| Category* | Citation of document, with indication, where appropriate, of the relevant passages | Relevant to claim No. |
| --- | --- | --- |
| PX | HK 1250580 A2 (MAGI-N MAGIC WATER HONG KONG LTD.) 28 December 2018 (2018-12-28) <br> claims 1-9 | 1-9 |
| A | CN 101888860 A (MICROONCOLOGY LTD.) 17 November 2010 (2010-11-17) <br> description, paragraphs 0017-0051 and 0099-0104 | 1-9 |
| A | CN 2727154 Y (YU, Huiyi) 21 September 2005 (2005-09-21) <br> entire document | 1-9 |
| A | CN 201411366 Y (SHENZHEN VIRTUE POWER TECHNOLOGY CO., LTD.) 24 February 2010 (2010-02-24) <br> entire document | 1-9 |
| A | CN 202961290 U (HAN, Chunlong) 05 June 2013 (2013-06-05) <br> entire document | 1-9 |
| A | CN 108224592 A (GUANGZHOU CHAOKAI TRADING CO., LTD.) 29 June 2018 (2018-06-29) <br> entire document | 1-9 |

☑ Further documents are listed in the continuation of Box C.      ☑ See patent family annex.

| | |
| --- | --- |
| \*    Special categories of cited documents: <br> "A"    document defining the general state of the art which is not considered to be of particular relevance <br> "D"    document cited by the applicant in the international application <br> "E"    earlier application or patent but published on or after the international filing date <br> "L"    document which may throw doubts on priority claim(s) or which is cited to establish the publication date of another citation or other special reason (as specified) <br> "O"    document referring to an oral disclosure, use, exhibition or other means <br> "P"    document published prior to the international filing date but later than the priority date claimed | "T"    later document published after the international filing date or priority date and not in conflict with the application but cited to understand the principle or theory underlying the invention <br> "X"    document of particular relevance; the claimed invention cannot be considered novel or cannot be considered to involve an inventive step when the document is taken alone <br> "Y"    document of particular relevance; the claimed invention cannot be considered to involve an inventive step when the document is combined with one or more other such documents, such combination being obvious to a person skilled in the art <br> "&"    document member of the same patent family |

| Date of the actual completion of the international search | Date of mailing of the international search report |
| --- | --- |
| **29 November 2019** | **05 December 2019** |

| Name and mailing address of the ISA/CN | Authorized officer |
| --- | --- |
| **China National Intellectual Property Administration (ISA/CN)** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2019/104746** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2000084566 A (TOTO LTD.) 28 March 2000 (2000-03-28)<br>    entire document | 1-9 |
| A | JP 2002113467 A (AISAN IND.) 16 April 2002 (2002-04-16)<br>    entire document | 1-9 |

Form PCT/ISA/210 (second sheet) (January 2015)

INTERNATIONAL SEARCH REPORT

Information on patent family members

International application No.

**PCT/CN2019/104746**

| Patent document cited in search report | | | Publication date (day/month/year) | Patent family member(s) | | | Publication date (day/month/year) |
|---|---|---|---|---|---|---|---|
| HK | 1250580 | A2 | 28 December 2018 | None | | | |
| CN | 101888860 | A | 17 November 2010 | EP | 2211915 | A1 | 04 August 2010 |
| | | | | US | 2010247403 | A1 | 30 September 2010 |
| | | | | CA | 2741135 | C | 20 October 2015 |
| | | | | US | 8696997 | B2 | 15 April 2014 |
| | | | | ES | 2568883 | T3 | 05 May 2016 |
| | | | | EP | 2211915 | B1 | 27 January 2016 |
| | | | | WO | 2009060214 | A1 | 14 May 2009 |
| | | | | CA | 2741135 | A1 | 14 May 2009 |
| | | | | CN | 101888860 | B | 17 July 2013 |
| CN | 2727154 | Y | 21 September 2005 | None | | | |
| CN | 201411366 | Y | 24 February 2010 | None | | | |
| CN | 202961290 | U | 05 June 2013 | None | | | |
| CN | 108224592 | A | 29 June 2018 | None | | | |
| JP | 2000084566 | A | 28 March 2000 | None | | | |
| JP | 2002113467 | A | 16 April 2002 | None | | | |

Form PCT/ISA/210 (patent family annex) (January 2015)

**EP 3 769 794 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- HK 18111547